# EUROPEAN PATENT APPLICATION

(11) **EP 2 177 235 A2**
(43) Date of publication of application: **21.04.2010**
(21) Application number: 09252416.4
(22) Date of filing: 15.10.2009
(51) Int. Cl.: A61L 17/14

(54) **Coating compositions**

(30) Priority: 15.10.2008 US 105480 P; 01.10.2009 US 571815
(71) Applicant: Tyco Healthcare Group LP, North Haven, CT 06473 (US)
(72) Inventor: Stopek, Joshua, Yalesville, CT 06492 (US); Hadba, Ahmad, Middlefield, CT 06455 (US)
(74) Representative: Soames, Candida Jane

(57) **Abstract**

The present disclosure provides compositions suitable for forming medical devices and coatings for medical devices, including sutures, which include a copolymer, at least one hydroxamate, and a fatty acid component. Sutures and other medical devices possessing such coatings are also provided.

## Description

### CROSS-REFERENCE TO RELATED APPLICATIONS

This application claims the benefit of and priority to U.S. Provisional Application Serial No. 61/105,480, filed October 15, 2008, the entire disclosure of which is incorporated by reference herein.

### TECHNICAL FIELD

Absorbable materials fabricated from compositions including caprolactone containing copolymers, hydroxamates, and a fatty acid component including fatty acids, salts of fatty acids, or salts of esters of fatty acids, are provided. In embodiments, the compositions of the present disclosure may be used to form medical devices or as coatings for surgical articles, including coatings for sutures.

### BACKGROUND

Synthetic absorbable multifilament sutures such as DEXON^{™}, VICRYL^{®}, and POLYSORB^{™}, commercially available from Ethicon, Inc. (Somerville, N.J.), and Tyco Healthcare Group LP, d\b\a\ Covidien (North Haven, CT), are known in the industry.

Suture coatings for synthetic absorbable sutures containing caprolactone are also known. See, for example, U.S. Patent Nos. 4,624,256; 4,190,720; 4,582,052; 4,605,730; 4,700,704; 4,705,820; 4,788,979; 4,791,929; 4,994,074; 5,047,048; 5,100,433; 5,133,739; and 5,352,515. Suture coatings containing esters of fatty acids and/or salts of fatty acids are also known. See, for example, U.S. Patent Nos. 5,716,376; 5,032,638; 4,711,241; 4,705,820; 4,201,216; and 4,027,676.

Matrix metalloproteinases (MMPs) are neutral zinc-dependent endopeptidases with substrate specificity for most extracellular matrix molecules, including collagens, gelatins, fibronectin, laminin and proteoglycan. They depend upon zinc for their catalytic activity.

Most cells do not express MMPs in vivo. Instead, growth factors, hormones, inflammatory cytokines, cell-matrix interactions and cellular transformation regulate their expression. Although the secretory granules of neutrophils and eosinophils are known to store some MMPs, most cell types normally synthesize very low quantities of MMPs.

MMPs share some common structural characteristics that include a signal sequence, an amino-terminal pro-peptide domain, a catalytic zinc binding domain, a proline-rich hinge region, and a carboxy-terminal hemopexin-like domain.

Extracellular matrix degradation is a normal event in the physiological remodeling associated with morphogenesis, reproduction, and in growth and maintenance processes such as cell migration, angiogenesis, and tissue regeneration. During inflammation and in several disease situations, however, excess MMPs may degrade the surrounding proteinaceous matrix, which may result in the destruction or weakening of connective tissue, unregulated cell migration/invasion, and/or tissue fibrosis. For example, connective tissue weakening or destruction may result in diseases such as rheumatoid arthritis, osteoarthritis, chronic periodontis, and arterial and cardiac aneurysm. Accordingly, MMP inhibitors have been used to treat osteoporosis, osteoarthritis, human chronic periodontal disease and various types of aneurysms.

Medical devices, including sutures, capable of reducing inflammation and preventing degradation of the extracellular matrix by MMPs, particularly in response to a disease or injury, remain desirable.

### SUMMARY

The present disclosure provides medical devices, coatings thereon, and processes for producing same. In embodiments, a coating for a medical device may include a copolymer comprising a predominant amount of epsilon-caprolactone and a minor amount of at least one other copolymerizable monomer, at least one hydroxamate, and a fatty acid component such as salts of a fatty acid and salts of a fatty acid ester.

In other embodiments, a medical device coating of the present disclosure may include a polymer including a lactone, and at least one hydroxamate of the formula: 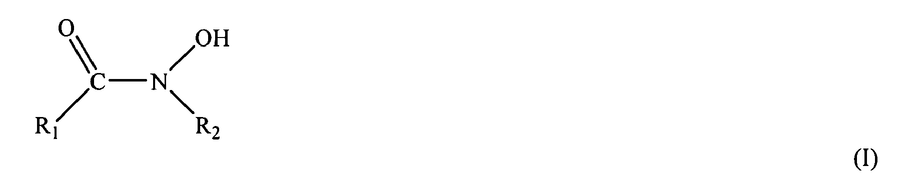 wherein R₁ is vinyl groups, acrylate groups, methacrylate groups, acid groups, alkyl groups, alkoxy groups, alkenyl groups, polymers terminated with the foregoing groups, and/or combinations thereof, and R₂ includes hydrogen.

In embodiments, medical devices which may be fabricated from, or possess a coating including, compositions of the present disclosure include sutures, surgical meshes, contact lenses, intraocular lenses, staples, clips, buttresses, lapbands, catheters, bandages, stents, grafts, stent/grafts, knotless wound closures, sealants, adhesives, anti-adhesion devices, anchors, tunnels, bone fillers, synthetic tendons, synthetic ligaments, grafts, tissue scaffolds, pins, screws, orthopedic hardware, pacers, pacemakers, drug delivery devices, soft tissue repair devices including mesh fixation, and implants.

In some embodiments, a suture of the present disclosure may include a fiber, a coating on at least a portion of the fiber, the coating including a copolymer including a predominant amount of epsilon-caprolactone and a minor amount of at least one other copolymerizable monomer, and at least one hydroxamate of the formula 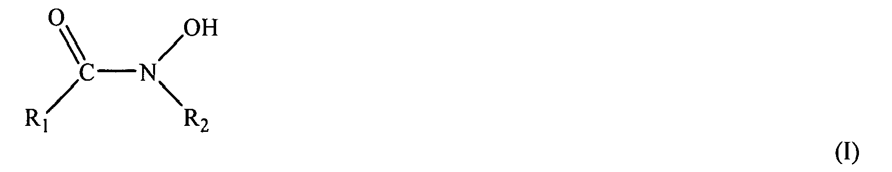 wherein R₁ is vinyl groups, acrylate groups, methacrylate groups, acid groups, alkyl groups, alkoxy groups, alkenyl groups, polymers terminated with the foregoing groups, and/or combinations thereof, and R₂ includes hydrogen.

### DETAILED DESCRIPTION

In embodiments, compositions useful in forming the aforementioned articles and coatings thereon include copolymers, hydroxamates, and optionally a fatty acid component. In some embodiments, the fatty acid component may be present as a predominant component. As used herein, a "predominant component" includes a component which is present in an amount greater than about 50 weight percent. Thus, a "minor component" includes a component which is present in an amount up to about 50 weight percent. In accordance with the present disclosure, where a composition of the present disclosure includes a fatty acid component as a predominant component, the minor component may be a combination of hydroxamates and copolymers.

In embodiments, the copolymers may include caprolactone. Suitable caprolactone containing copolymers include copolymers which may be synthesized by polymerization techniques within the purview of those skilled in the art. In embodiments, the caprolactone containing copolymer may be obtained by polymerizing a major amount of epsilon-caprolactone and a minor amount of at least one other copolymerizable monomer or mixture of such monomers in the presence of a polyhydric alcohol initiator. In other embodiments, caprolactone containing copolymers which may be utilized include "star" copolymers obtained by polymerizing a major amount of epsilon-caprolactone and a minor amount of another bioabsorbable monomer polymerizable therewith, optionally in the presence of a polyhydric alcohol initiator.

Suitable monomers which can be copolymerized with epsilon-caprolactone include alkylene carbonates such as trimethylene carbonate, tetramethylene carbonate, dimethyl trimethylene carbonate; dioxanones; dioxepanones; absorbable cyclic amides; absorbable cyclic ether-esters derived from crown ethers; hydroxyacids capable of esterification, including both alpha hydroxy acids (such as glycolic acid and lactic acid) and beta hydroxyacids (such as beta hydroxybutyric acid and gamma hydroxyvaleric acid); polyalkyl ethers (such as polyethylene glycol and polypropylene glycol and combinations thereof); glycolides and combinations thereof.

Suitable polyhydric alcohol initiators which may be utilized in forming the caprolactone containing copolymers include glycerol; trimethylolpropane; 1,2,4-butanetriol; 1,2,6-hexanetriol; triethanolamine; triisopropanolamine; erythritol; threitol; pentaerythritol; ribitol; arabinitol; xylitol; N,N,N',N'-tetrakis(2-hydroxyethyl)ethylenediamine; N,N,N',N'-tetrakis(2-hydroxypropyl)ethylenediamine; dipentaerythritol; allitol; dulcitol; glucitol; altritol; iditol; sorbitol; mannitol; inositol; and the like; with mannitol being useful in some embodiments.

The polyhydric alcohol initiator may be generally employed in small amounts, e.g., from about 0.01 to about 5 weight percent of the total monomer mixture, in embodiments from about 0.1 to about 3 weight percent of the total monomer mixture.

The polymerization of the above monomers with caprolactone contemplates all of the various types of monomer addition, for example, simultaneous, sequential, simultaneous followed by sequential, sequential followed by simultaneous, and the like.

The caprolactone containing copolymers can contain from about 70 to about 98 weight percent epsilon-caprolactone derived units, in embodiments from about 80 to about 95 weight percent epsilon-caprolactone derived units, the balance of the copolymer being derived from the other copolymerizable monomer(s).

In embodiments, the copolymerizable monomer may be glycolide, optionally in combination with other monomer(s).

In other embodiments, any of the monomers identified above as suitable as copolymerizable with epsilon caprolactone may be utilized without caprolactone; i.e., they may be used as homopolymers or combined with monomers other than caprolactone to form a polymer for use in accordance with the present disclosure. Similarly, in embodiments, caprolactone may be utilized as a homopolymer. Thus, in embodiments, polymers utilized to form a composition of the present disclosure may include a lactone. Suitable lactones include, for example, polylactic acid, polyglycolic acid, dioxanone, epsilon-caprolactone, trimethylene carbonate, alone or in combination with other monomers.

In yet other embodiments, the copolymers utilized in accordance with the present disclosure may be made at least in part from a polyoxyalkylene block copolymer. Suitable polyoxyalkylene block copolymers include those having an A-B or A-B-A structure wherein "A" is a block made from repeating units of the formula -O(CH₂)ₙ- where n is from 1 to 4 and "B" is a block made from repeating units that are different from the repeating units in the A block and are selected from groups of the formula -O(CH₂)ₙ- where n is from 1 to 4. In particularly useful embodiments, a co-polymer designated as "PEO-PPO-PEO", wherein "PEO" denotes a block of repeating units of the formula - OCH₂CH₂- and "PPO" denotes a block of repeating units of the formula -OCH₂CH₂CH₂-. Particularly useful are triblock copolymers of the formula HO(C₂H₄O)ₐ(C₃H₆O)_{b}(C₂H₄O)_{c}H wherein a and c are independently from 1-150 units and b ranges from 10-200 units, with the overall molecular weight ranging from 1,000 to 50,000 daltons. Such polyoxyalkylene block copolymers may be, in embodiments, referred to by those skilled in the art as "poloxamers". Suitable poloxamers include those where a equals c and b ranges from 10-200 units.

Examples of polyoxyalkylene block copolymers which may be utilized to form the copolymer utilized in forming the compositions of the present disclosure include poloxamers sold under the trade names PLURONIC^{®} (BASF Corp.) or SYNPERONIC^{®} (ICI). PLURONIC^{®} copolymers are identified by a specific letter-number combination. The alphabetical designation describes the physical form of the product: 'L' for liquids, 'P' for pastes, 'F' for solid forms. The first digit (two digits in a three-digit number) in the numerical designation, multiplied by 300, indicates the approximate molecular weight of the hydrophobic component (propylene oxide). The last digit, when multiplied by 10, indicates the approximate hydrophilic (ethylene oxide) content of the molecule as a percentage by weight. Thus, for example, PLURONIC^{®} F68 is a solid material. The molecular weight of the hydrophobic (propylene oxide) component is approximately 1800 (6 X 300). The hydrophilic (ethylene oxide) component represents approximately 80% of the molecule by weight (8 X 10).

Poloxamers can be roughly divided into 3 main categories, each of which may be useful in making the copolymer component of the compositions of the present disclosure, namely emulsion forming, micelle forming, and water soluble poloxamers. Various factors which determine poloxamer characteristics and behavior include the molecular weight, PPO:PEO ratio, temperature conditions, concentration, and presence of ionic materials. There is thus a wide range of characteristics in existing commercially available poloxamers which can be exploited in formulating the compositions of the present disclosure, especially where the composition further includes a medicinal agent or other bioactive agent.

In embodiments, a suitable poloxamer which may be utilized in the copolymer component of a composition of the present disclosure includes a polyoxyethylene-polyoxypropylene triblock copolymer known as poloxamer 188, sold under the trade name PLURONIC^{®} F68 by BASF (Parsippany, N.J.). Other poloxamers which may be utilized in the compositions of the present disclosure include poloxamer 403 (sold as PLURONIC^{®} P123), poloxamer 407 (sold as PLURONIC^{®} P127), poloxamer 402 (sold as PLURONIC^{®} P122), poloxamer 181 (sold as PLURONIC^{®} L61), poloxamer 401 (sold as PLURONIC^{®} L121), poloxamer 185 (sold as PLURONIC^{®} P65), and poloxamer 338 (sold as PLURONIC^{®} F108).

The polyoxyalkylene block copolymers may, in some embodiments, be reacted with additional biocompatible, biodegradable monomers to form the copolymer. Suitable monomers which may be reacted with the polyoxyalkylene block copolymers include, for example, alpha-hydroxy acids, lactones, carbonates, esteramides, anhydrides, amino acids, orthoesters, alkylene alkylates, alkylene oxides, biodegradable urethanes, and combinations thereof. Specific examples of suitable biocompatible, biodegradable monomers which may be added to the poloxamer include glycolide, lactide, hydroxybutyric acid, hydroxyvaleric acid, caprolactone, trimethylene carbonate, dimethyl trimethylene carbonate, p-dioxanone, and combinations thereof. These monomers, alone or in combination, can constitute up to about 90% to by total weight of the copolymer component, in embodiments from about 10% to about 75% by total weight of the copolymer component, in other in embodiments from about 30% to about 65% by total weight of the copolymer component, with the polyoxyalkylene block copolymer making up the balance of the copolymer component. It should, of course, be understood that the other monomers may be reacted first to form a polymer (homopolymer or copolymer (e.g., random, block or the like)) prior to reaction with the polyoxyalkylene block copolymer. Conditions suitable for conducting such reactions are within the purview of those skilled in the art.

In other embodiments, a polyoxyalkylene block copolymer may be reacted with a monomer mixture that includes a major amount of epsilon-caprolactone and a minor amount of at least one other copolymerizable monomer or mixture of such monomers in the presence of a polyhydric alcohol initiator as disclosed in U.S. Patent No. 6,177,094. Such monomer mixtures may also be utilized without the polyalkylene block copolymers. The polymerization of the above monomers contemplates all of the various types of monomer addition, i.e., simultaneous, sequential, simultaneous followed by sequential, sequential followed by simultaneous, etc. Suitable monomers which can be copolymerized with epsilon-caprolactone include glycolide, lactide, p-dioxanone and trimethylene carbonate.

In embodiments, an ε-caprolactone polymer containing a major amount of epsilon-caprolactone and a minor amount of at least one other copolymerizable monomer or mixture of such monomers, including the caprolactone copolymers described above in combination with a copolymerizable monomer such as glycolide, may be reacted with a polyoxyalkylene block copolymer.

In embodiments, a suitable copolymer for use in accordance with the present disclosure includes one possessing caprolactone units present in an amount greater than about 50% by weight, in embodiments from about 50% by weight to about 55% by weight; glycolide units present in an amount from about 5% to about 15% by weight; and a polyoxyethylene-polyoxypropylene triblock copolymer, in embodiments poloxamer 188 described above (sold under the trade name PLURONIC^{®} F68 by BASF (Parsippany, N.J.)), in an amount from about 30% to about 45% by weight.

The compositions of the present disclosure may also possess at least one hydroxamate. Suitable hydroxamates for use in the compositions of the present disclosure include components possessing a hydroxamate group of the following formula (I): 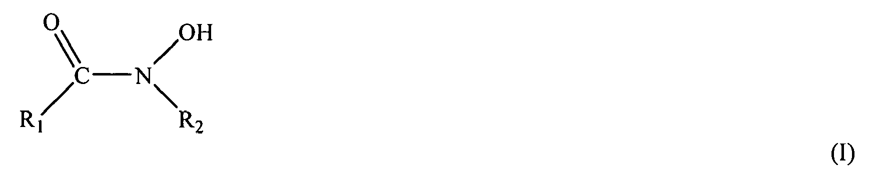 wherein R₁ may include vinyl groups, including vinyl alcohols such as polyvinyl alcohol, acrylate groups including alkyl acrylates, methacrylate groups including alkyl methacrylates, hydroxyethyl methacrylates, acid groups including acrylic acid, methacrylic acid, other alkyl groups, alkoxy groups, alkenyl groups, and polymers possessing hydroxyl groups, including polymers terminated with any of the above groups, such as vinyl alcohols, hydroxyethyl methacrylates, and the like, and R₂ may be hydrogen.

As used herein, "alkyl", used either alone or in compound words such as "haloalkyl" or "alkylthio", includes straight chain or branched C₁₋₁₂ alkyl groups. Examples include methyl, ethyl, propyl, isopropyl and the like.

As used herein, "alkoxy" includes straight chain or branched alkoxy, in embodiments C₁₋₁₂ alkoxy such as methoxy, ethoxy, n-propoxy, isopropoxy and butoxy isomers.

As used herein, "alkenyl" includes groups formed from straight chain, branched or mono- or polycyclic alkenes including ethylenically mono- or poly-unsaturated alkyl or cycloalkyl groups as previously defined, in embodiments C₂₋₁₂. alkenyl. Examples of alkenyl include vinyl; allyl; 1-methylvinyl; butenyl; iso-butenyl; 3-methyl-2-butenyl; 1-pentenyl; cyclopentenyl; 1-methyl-cyclopentenyl; 1-hexenyl; 3-hexenyl; cyclohexenyl; 1-heptenyl; 3-heptenyl; 1-octenyl; cyclooctenyl; 1-nonenyl; 2-nonenyl; 3-nonenyl; 1-decenyl; 3-decenyl; 1,3-butadienyl; 1-4,pentadienyl; 1,3-cyclopentadienyl; 1,3-hexadienyl; 1,4-hexadienyl; 1,3-cyclohexadienyl; 1,4-cyclohexadienyl; 1,3-cycloheptadienyl; 1,3,5-cycloheptatrienyl; or 1,3,5,7-cyclooctatetraenyl.

Additional monomers and comonomers which may be used, in some embodiments, to form the hydroxamates include, for example, (alk)acrylates, (alk)acrylics and (alk)acrylamides, which may mean acrylate or alkacrylate, acrylic or alkacrylic and acrylamide or alkacrylamide respectively. Unless otherwise stated, alkacrylate, alkacrylic and alkacrylamide groups may contain from about 1 to about 4 carbon atoms in the alkyl group thereof and may be methacrylate, methacrylic or methacrylamide groups. Similarly (meth)acrylate, (meth)acrylic and (meth)acrylamide shall be understood to mean acrylate or methacrylate, acrylic or methacrylic and acrylamide or methacrylamide respectively.

Methods for forming these hydroxamate functional components are within the purview of those skilled in the art. For example, in embodiments, a hydroxamate functional polymer may be produced by the surface modification of cross-linked polymethacrylic acid (PMAA)-co-methyl methacrylate (MAA) beads, thus producing a hydroxamate functional polymer, i.e., PMAA-MMA-hydroxamate as the hydroxamate functional component.

In embodiments, polymerizable hydroxamate monomers may be copolymerized with the copolymers described above, for example the caprolactone containing copolymers, or blended therewith. The hydroxamate monomer, which may be encompassed by formula I above, may have an R₁ including CH₂=C-CH₃, and R₂ may be hydrogen. In other embodiments, the hydroxamate monomer may be utilized to synthesize a hydroxamate homopolymer, or may be copolymerized with any other suitable comonomers to produce copolymers which, in turn, may be copolymerized with the copolymers described above, for example the caprolactone containing copolymers, or blended therewith.

Hydroxamate homopolymers synthesized from the above hydroxamate monomer can also be grafted onto any derivatizable polymer. The resulting hydroxamate functional composition, whether a monomer, homopolymer, or copolymer, may then be combined with the copolymers described above, for example a caprolactone containing copolymer, either as a comonomer or as a blend.

It should, of course be understood that two or more hydroxamates may be utilized in forming compositions of the present disclosure.

Conditions for conducting polymerization are within the purview of those skilled in the art and include those described above for the polymerization of the caprolactone copolymer. In other embodiments, polymerization may be initiated by subjecting the monomers, for example, the monomers utilized in forming the caprolactone copolymer, and the hydroxamate, to energy including irradiation, such as high energy radiation including gamma and/or e-beam, ultraviolet light, pulse laser ablation deposition, plasma energy treatment, chemical initiation, photoinitiation, and the like. In embodiments, the use of high energy radiation initiation may be beneficial as it should not require the use of an additional initiator such as a chemical initiator or catalyst.

Other conventional techniques may be used for polymerization, including thermal or photochemical polymerization. Where comonomers capable of producing crosslinking in the coated polymer film are present, the polymerization conditions may be set such that crosslinking does not occur during polymerization. Thus, for example, actinic radiation may not be used to prepare a polymer containing a comonomer which can form crosslinks by exposure to actinic radiation.

For thermal polymerization, a temperature of from about 40°C to about 100°C, in embodiments from about 50°C to about 80°C, may be used. For photochemical polymerization actinic radiation such as gamma, ultraviolet (UV), visible, or microwave radiation may be used. In some embodiments, UV radiation of a wavelength from about 200 nm to about 400 nm may be used.

The polymerization is generally performed in a reaction medium, which is for instance a solution or dispersion using as a solvent for example acetonitrile, dimethyl formamide, chloroform, dichloromethane, methylene chloride, ethyl acetate, dimethyl sulfoxide, dioxane, benzene, toluene, tetrahydrofuran, or where the polymer does not contain groups which react with protic solvents, water or an alkanol containing from about 1 to about 4 carbon atoms, e.g., methanol, ethanol or isopropanol. Alternatively, a mixture of any of the above solvents may be used.

In embodiments, the components of a composition of the present disclosure, for example the lactone polymer and/or the hydroxamates, may be emulsified, dispersed, and/or mixed in the above solvents to form a suitable coating composition of the present disclosure.

The polymerization may be carried out in the presence of one or more polymerization initiators, including polyhydric alcohols as described above.

Generally the polymerization is performed for a period from about 1 hour to about 72 hours, in embodiments from about 8 hours to about 48 hours, in some cases from about 16 hours to about 24 hours, and under an inert atmosphere of for example nitrogen or argon. The polymer may be purified by dialysis, precipitation in a non-solvent (e.g., diethyl ether or acetone) or ultrafiltration. The resulting polymer is generally dried under vacuum, e.g., for a period of time from about 5 hours to about 72 hours and has a molecular weight from about 10,000 to about 10 million, in embodiments from about 20,000 to about 1 million.

The composition of the present disclosure may thus contain an initiator and/or any other components such as a chain transfer agent, acid, base, surfactant, emulsifier or catalyst of conventional type, each in an amount from about 0.1% to about 5%, in embodiments from about 0.2% to about 3% and in some cases about 0.5%, by weight each relative to the total weight of the monomers.

In addition to the copolymer(s) and hydroxamate(s) described above, compositions in accordance with the present disclosure may also include a fatty acid component, such as a fatty acid or a fatty acid salt or a salt of a fatty acid ester. Suitable fatty acids may be saturated or unsaturated, and may include higher fatty acids having more than about 12 carbon atoms. Suitable saturated fatty acids include, for example, stearic acid, palmitic acid, myristic acid and lauric acid. Suitable unsaturated fatty acids include oleic acid, linoleic acid, and linolenic acid. In addition, an ester of fatty acids, such as sorbitan tristearate or hydrogenated castor oil, may be used.

Suitable fatty acid salts include the polyvalent metal ion salts of C₆ and higher fatty acids, particularly those having from about 12 to about 22 carbon atoms, and mixtures thereof. Fatty acid salts including the calcium, magnesium, barium, aluminum, and zinc salts of stearic, palmitic and oleic acids may be useful in some embodiments of the present disclosure, that is, calcium stearate, magnesium stearate, barium stearate, aluminum stearate, zinc stearate, calcium palmitate, magnesium palmitate, barium palmitate, aluminum palmitate, zinc palmitate, calcium oleate, magnesium oleate, barium oleate, aluminum oleate, and zinc oleate. Some useful salts include commercial "food grade" calcium stearate which contains a mixture of about one-third C₁₆ and two-thirds C₁₈ fatty acids, with small amounts of the C₁₄ and C₂₂ fatty acids.

Suitable salts of fatty acid esters which may be included in the compositions of the present disclosure include salts of lactylate esters of C₁₀ or greater fatty acids. Exemplary salts of fatty acid esters include, for example, calcium, magnesium, aluminum, barium, or zinc stearoyl lactylate; calcium, magnesium, aluminum, barium, or zinc palmityl lactylate; and/or calcium, magnesium, aluminum, barium, or zinc oleyl lactylate. In embodiments calcium stearoyl-2-lactylate (such as the calcium stearoyl-2-lactylate commercially available under the tradename VERV from American Ingredients Co., Kansas City, Mo.) may be utilized. Other fatty acid ester salts which may be utilized include those selected from the group consisting of lithium stearoyl lactylate, potassium stearoyl lactylate, rubidium stearoyl lactylate, cesium stearoyl lactylate, francium stearoyl lactylate, sodium palmityl lactylate, lithium palmityl lactylate, potassium palmityl lactylate, rubidium palmityl lactylate, cesium palmityl lactylate, francium palmityl lactylate, sodium oleyl lactylate, lithium oleyl lactylate, potassium oleyl lactylate, rubidium oleyl lactylate, cesium oleyl lactylate, and francium oleyl lactylate.

As noted above, the hydroxamates may be combined with the copolymer(s) and, together, they may form the minor component of the composition of the present disclosure, while the fatty acid component may form the predominant component of the composition of the present disclosure. Thus, in embodiments, the copolymers may be present in the compositions of the present disclosure in amounts from about 1 to about 9 percent by weight of the composition, in embodiments from about 2 to about 7 percent by weight of the composition. The hydroxamates may be present in amounts from about 0.5 to about 9 percent by weight of the composition, in embodiments from about 1 to about 5 percent by weight of the composition. The fatty acid component may thus be present in amounts from about 0.5 to about 5 percent by weight of the composition, in other embodiments from about 1 to about 3 percent by weight of the composition.

The copolymer, the fatty acid component, and the hydroxamate are non-toxic; a combination of the three is non-toxic as well.

The compositions of the present disclosure including hydroxamates may be used as coatings for medical devices and implants. Chronic wounds may take months or years to heal due, in part, to high levels of MMPs that degrade the newly formed matrix even as it is synthesized. The coatings of the present disclosure including hydroxamates, due to the presence of the hydroxamate group, may inhibit the activity of the MMPs in or adjacent a wound, thereby promoting healing.

Similarly, angiogenesis or vasculogenesis of tumors and the formation of metastases require cell migration and invasion, which are enabled by the release of pro-MMPs. The coatings of the present disclosure including hydroxamates, which counteract those MMPs, may thus be suitable for minimizing angiogenesis and/or vascularization of tumors.

Furthermore tissue remodeling occurs secondary to secretion or expression of MMP's. Thus blood vessels associated with wound repair are resorbed or ischemic tissue are destroyed by MMP action. The coatings of the present disclosure including hydroxamates, which counteract those MMPs, may thus be suitable to enhance would repair.

The activity of MMPs is also essential for many of the processes involved in atherosclerotic plaque formation (infiltration of inflammatory cells, angiogenesis, and smooth muscle cell migration and proliferation). Elevated levels of MMPs are expressed in human atherosclerotic plaque and at the sites of aneurysm. Furthermore, matrix degradation by MMPs may cause the plaque instability and rupture that leads to the clinical symptoms of atherosclerosis. The coatings of the present disclosure including hydroxamates, which counteract those MMPs, may thus be suitable to reduce the formation of atherosclerotic plaques and the incidence of rupture at the sites of aneurysm.

There is also accumulating evidence that an increase in the proportion of active MMPs is associated with the progression of restenosis following vascular interventions such as balloon angioplasty or intra-coronary stenting, for the treatment of coronary artery disease. In contrast to the non-diseased vessel wall, which constitutively expresses only pro-(inactive) MMP-2, injured or atherosclerotic arteries demonstrate a dramatic increase in MMP-2 activity. This occurs in conjunction with induced expression of MMPs-3, -7, -9, - 12, and -13. The coatings of the present disclosure including hydroxamates, which counteract those MMPs, may thus be utilized to reduce restenosis.

The coatings of the present disclosure including hydroxamates may inactivate MMPs by binding the zinc at the active center of the enzymes. With multiple point attachments, the hydroxamates behave like a molecular magnet for zinc.

In embodiments, the compositions of the present disclosure possessing hydroxamates may bind to the active form of MMPs, without and specificity for particular MMP types. In other embodiments, the compositions of the present disclosure possessing hydroxamates may provide preferential binding to active forms of MMPs in the local tissue environment. This may be advantageous because it specifically targets one stage in the MMP regulatory cascade, namely that directly preceding matrix degradation. In addition, selective binding may reduce the risk of over inhibition which would delay healing by preventing a healthy rate of tissue turnover and essential processes such as cell migration and angiogenesis.

The compositions of the present disclosure may release low concentrations of the hydroxamate, thus providing inhibition of MMP activity where the hydroxamate is released at the site of implantation or injury to which the composition of the present disclosure is applied.

Methods for combining the copolymers, hydroxamates, and fatty acid components to form compositions of the present disclosure are within the purview of those skilled in the art and include, for example, mixing, blending, and the like. In addition to blends, as noted above, in some embodiments the copolymer may include the hydroxamate, which may then be combined with the fatty acid component.

In embodiments, a fatty acid ester such as calcium stearoyl lactylate may be utilized in forming a composition of the present disclosure. Since such fatty acid esters are soluble, coatings fabricated from compositions of the present disclosure including such fatty acid esters, hydroxamates, and copolymers, including the caprolactone containing copolymers described above, may be applied as solutions to articles including surgical articles by processes including spraying, dipping, and the like.

In embodiments, the compositions of the present disclosure may be applied in a solution utilizing any suitable solvent within the purview of those skilled in the art. Such solvents include, for example, methylene chloride, chloroform, N,N-dimethylformamide, N-methylpyrrolidone, methanol, ethanol, n-propanol, isopropanol, hexane, heptane, cyclohexane, tetrahydrofuran (THF), dipropyl ether, dioxolane, methyl ethyl ketone, methyl isobutyl ketone, toluene, xylene, acetonitrile, ethyl acetate, butyl acetate, combinations thereof, and the like.

In embodiments, coating suspensions may be utilized wherein the composition of the present disclosure is combined with multiple solvents, including any solvent described above. In embodiments, suitable solvents for forming coating suspensions include, but are not limited to: lower alcohols including those having from about 1 to about 6 carbon atoms such as methanol, ethanol, n-propanol, isopropanol (IPA), n-butanol, combinations thereof, and the like; hexanes including hexane, cyclohexane, combinations thereof, and the like; chlorinated solvents such as methylene chloride, chloroform, chlorobenzene, 1,2-dichloroethane (also known as ethylene dichloride), dichlorobenzene, combinations thereof, and the like. As noted above, in some embodiments multiple solvents may be utilized, including any combination of the foregoing solvents.

For example, the composition of the present disclosure can be applied as a coating by any suitable process, for example, by passing a medical device through a solution of the composition, past a brush or other coating solution applicator, or past one or more spray nozzles dispensing a solution possessing a composition of the present disclosure for use as a coating. The article wetted with the coating solution may then be subsequently passed through, or held in, a drying oven for a time and at a temperature sufficient to vaporize and drive off the solvent. If desired, the coating composition can optionally contain additional components, e.g., dyes, antibiotics, antiseptics, growth factors, anti-inflammatory agents, and the like.

Where applied as a coating, the composition of the present disclosure may be applied in a continuous, that is, a single step. In other embodiments, the composition of the present disclosure may be applied in discontinuous or semi-continuous steps, that is, a multi-step process.

The composition of the present disclosure may be applied as a coating in a single layer or, in embodiments, the composition of the present disclosure may be applied as a coating in multiple layers and/or as overcoats to other preexisting coatings on an article. In some embodiments, a coating of the present disclosure may include the three components described herein applied sequentially. For example, a copolymer, such as a caprolactone containing copolymer, may be combined with a fatty acid component, and the combination of the two may then be utilized to form a medical device or a coating thereon. The hydroxamates described above may then be applied as a separate coating to the device made from, or already possessing a coating including, the caprolactone containing copolymer combined with the fatty acid component.

The compositions of the present disclosure may find many uses in the formation of medical devices and coatings thereon. In embodiments, surgical articles can be manufactured from, or coated with, the compositions described herein. Suitable medical devices include, but are not limited to, clips and other fasteners, staples, sutures, pins, screws, prosthetic devices, wound dressings, bandages, drug delivery devices, anastomosis rings, surgical blades, contact lenses, intraocular lenses, surgical meshes, stents, stent coatings, grafts, catheters, stent/grafts, knotless wound closures, sealants, adhesives, contact lenses, intraocular lenses, anti-adhesion devices, anchors, tunnels, bone fillers, synthetic tendons, synthetic ligaments, tissue scaffolds, stapling devices, buttresses, lapbands, orthopedic hardware, pacers, pacemakers, and other implants and implantable devices.

Fibers can be made from, or coated with, the compositions of the present disclosure. In embodiments, fibers made or coated with the compositions of the present disclosure may be knitted or woven with other fibers, either absorbable or non-absorbable fibers, to form textiles. The fibers also can be made into non-woven materials to form fabrics, such as meshes and felts.

In embodiments, the fatty acid component combined with hydroxamates and copolymers can advantageously be mixed to form a composition (with the fatty acid component as the predominant component thereof) useful in coating surgical sutures.

While the coating composition herein can be applied to any type of suture, it may, in some embodiments, be useful for coating a multi-filament suture, in embodiments a braided suture, including those disclosed in U.S. Patent No. 5,019,093, the entire disclosure of which is incorporated by reference herein. In embodiments, useful compositions for coating braided multifilament sutures may include at least about 52 percent fatty acid component, the remainder being the hydroxamate in combination with the copolymer. Applied to a suture, the coating composition results in advantageous improvement in one or more properties of the suture, e.g., knot security, surgeon's throw, lubricity, knot run down, and/or knot repositioning.

The amount of coating composition applied to a braided suture will vary depending upon the structure of the suture, e.g., the number of filaments, tightness of braid or twist, the size of the suture, and its composition. Suitable coating levels range from about 0.3% to about 10% by weight of the suture, with about 0.5% to about 5% by weight of the suture being useful in some embodiments.

The coated suture may be attached to a surgical needle by methods within the purview of those skilled in the art. Wounds may be sutured by passing the needled suture through tissue to create wound closure. The needle may then be removed from the suture and the suture tied. The coating advantageously enhances the surgeon's ability to pass the suture through tissue as well as to increase the ease and security with which he/she can tie the suture.

Sutures possessing coatings of the present disclosure possess excellent handling properties and knot security, which may, in embodiments, be determined using tie board testing and similar methods within the purview of those skilled in the art. Sutures possessing coatings of the present disclosure also possess excellent knot rundown and reposition, and both wet and dry handling characteristics. Methods for determining these properties are within the purview of those skilled in the art, including the use of apparatus and methods from Instron Corporation (Norwood, MA).

It will be appreciated that various of the above-disclosed and other features and functions, or alternatives thereof, may be desirably combined into many other different systems or applications. Also that various presently unforeseen or unanticipated alternatives, modifications, variations or improvements therein may be subsequently made by those skilled in the art which are also intended to be encompassed by the following claims. Unless specifically recited in a claim, steps or components of claims should not be implied or imported from the specification or any other claims as to any particular order, number, position, size, shape, angle, color, or material.

## Claims

1. A coating for a medical device comprising:
a copolymer comprising a predominant amount of epsilon-caprolactone and a minor amount of at least one other copolymerizable monomer;
at least one hydroxamate; and
a fatty acid component selected from the group consisting of salts of a fatty acid and salts of a fatty acid ester.

2. The coating of claim 1, wherein the copolymer comprises from about 70 to about 98 weight percent epsilon-caprolactone derived units; or the coating of claim 1, wherein the copolymer comprises from about 80 to about 95 weight percent epsilon-caprolactone derived units.

3. The coating of claim 1 or claim 2, wherein the at least one other copolymerizable monomer comprises glycolide.

4. The coating of any one of the preceding claims, wherein the at least one hydroxamate is of the formula: 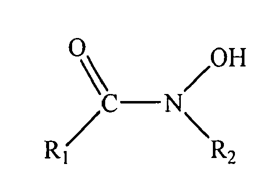 wherein R₁ is selected from the group consisting of vinyl groups, acrylate groups, methacrylate groups, acid groups, alkyl groups, alkoxy groups, alkenyl groups, polymers terminated with the foregoing groups, and combinations thereof, and R₂ comprises hydrogen; preferably wherein R₁ is selected from the group consisting of alkyl acrylates, alkyl methacrylates, acrylic acid, methacrylic acid, and combinations thereof.

5. The coating of any one of the preceding claims, wherein the salt of a fatty acid comprises a polyvalent metal ion salt of C₆ and higher fatty acids; preferably wherein the salt of a fatty acid is selected from the group consisting of calcium stearate, magnesium stearate, barium stearate, aluminum stearate, zinc stearate, calcium palmitate, magnesium palmitate, barium palmitate, aluminum palmitate, zinc palmitate, calcium oleate, magnesium oleate, barium oleate, aluminum oleate, and zinc oleate.

6. The coating of any one of the preceding claims, wherein the salt of a fatty acid ester comprises a salt of lactylate esters of C₁₀ or greater fatty acids; preferably wherein the salt of lactylate esters of C₁₀ or greater fatty acids is selected from the group consisting of calcium stearoyl lactylate, magnesium stearoyl lactylate, aluminum stearoyl lactylate, barium stearoyl lactylate, zinc stearoyl lactylate, calcium palmityl lactylate, magnesium palmityl lactylate, aluminum palmityl lactylate, barium palmityl lactylate, zinc palmityl lactylate, calcium oleyl lactylate, magnesium oleyl lactylate, aluminum oleyl lactylate, barium oleyl lactylate, and zinc oleyl lactylate.

7. The coating of any one of the preceding claims, further comprising a solvent selected from the group consisting of methylene chloride, chloroform, N,N-dimethylformamide, N-methylpyrrolidone, methanol, ethanol, n-propanol, isopropanol, hexane, heptane, cyclohexane, tetrahydrofuran, dipropyl ether, dioxolane, methyl ethyl ketone, methyl isobutyl ketone, toluene, xylene, acetonitrile, ethyl acetate, butyl acetate, and combinations thereof.

8. The coating of any one of the preceding claims, wherein the components of the coating are applied separately in multiple layers; or the coating of any one of the preceding claims, wherein the components of the coating are applied as a single layer.

9. The coating of any one of the preceding claims, wherein the medical device is selected from the group consisting of sutures, surgical meshes, contact lenses, intraocular lenses, staples, clips, buttresses, lapbands, catheters, bandages, stents, grafts, stent/grafts, knotless wound closures, sealants, adhesives, anti-adhesion devices, anchors, tunnels, bone fillers, synthetic tendons, synthetic ligaments, grafts, tissue scaffolds, pins, screws, orthopedic hardware, pacers, pacemakers, drug delivery devices, soft tissue repair devices, and implants.

10. A mesh possessing the coating of claim 1, wherein the coating is present in an amount from about 0.3% to about 10% by weight of the medical device.

11. A medical device coating comprising:
a polymer comprising a lactone; and
at least one hydroxamate of the formula: 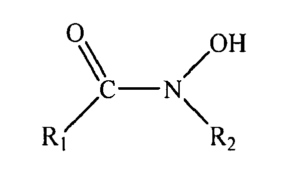
wherein R₁ is selected from the group consisting of vinyl groups, acrylate groups, methacrylate groups, acid groups, alkyl groups, alkoxy groups, alkenyl groups, polymers terminated with the foregoing groups, and combinations thereof, and R₂ comprises hydrogen.

12. The medical device of claim 11, wherein the lactone is selected from the group consisting of polylactic acid, polyglycolic acid, dioxanone, epsilon-caprolactone, trimethylene carbonate, and combinations thereof.

13. The medical device of claim 11 or claim 12, further comprising a solvent selected from the group consisting of acetonitrile, dimethyl formamide, chloroform, dichloromethane, methylene chloride, ethyl acetate, dimethyl sulfoxide, dioxane, benzene, toluene, tetrahydrofuran, water, methanol, ethanol, isopropanol, and combinations thereof.

14. The medical device of any one of claims 11 to 13, wherein the medical device is selected from the group consisting of sutures, surgical meshes, contact lenses, intraocular lenses, staples, clips, buttresses, lapbands, catheters, bandages, stents, grafts, stent/grafts, knotless wound closures, sealants, adhesives, anti-adhesion devices, anchors, tunnels, bone fillers, synthetic tendons, synthetic ligaments, grafts, tissue scaffolds, pins, screws, orthopedic hardware, pacers, pacemakers, and implants.

15. A suture comprising:
a fiber;
a coating on at least a portion of the fiber, the coating comprising:
a copolymer comprising a predominant amount of epsilon-caprolactone and a minor amount of at least one other copolymerizable monomer; and
at least one hydroxamate of the formula: 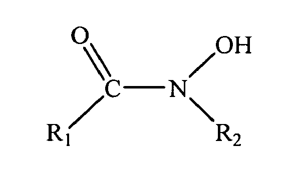
wherein R₁ is selected from the group consisting of vinyl groups, acrylate groups, methacrylate groups, acid groups, alkyl groups, alkoxy groups, alkenyl groups, polymers terminated with the foregoing groups, and combinations thereof, and R₂ comprises hydrogen.
